# FASCICULE DE BREVET EUROPEEN

(11) **EP 1 241 156 B1**
(45) Date de publication et mention de la délivrance du brevet: **18.08.2004**
(21) Numéro de dépôt: 02290080.7
(22) Date de dépôt: 14.01.2002
(51) Int. Cl.: C07C 45/50, C07C 45/80

(54) **Procédé amélioré pour l'hydroformylation de composés oléfiniquement insaturés dans un solvant ionique non-aqueux**
Verfahren zur Hydroformylierung von olefinisch ungesättigten Verbindungen in einer nicht-wässrigen ionischen Flüssigkeit
Process for the hydroformylation of olefinic unsaturated compounds in a non-aqueous ionic solvent

(30) Priorité: 24.01.2001 FR 0100970
(43) Date de publication de la demande: 18.09.2002
(73) Titulaire: Institut Français du Pétrole, 92852 Rueil-Malmaison Cedex (FR)
(72) Inventeur: Favre, Frédéric, 69190 Saint-Fons (FR); Commereuc, Dominique, 92190 Meudon (FR); Olivier-Bourbigou, Hélène, 92500 Rueil-Malmaison (FR)

(56) Documents cités:
- EP-A- 0 776 880
- EP-A- 1 106 595
- DE-A- 19 919 495

## Description

La présente invention concerne un procédé amélioré d'hydroformylation de composés oléfiniquement insaturés.

L'hydroformylation des composés oléfiniques est une réaction de grande importance industrielle et la plupart des procédés ont recours à des catalyseurs homogènes dissous dans une phase organique constituée des réactifs, des produits et éventuellement d'un excès de ligand, si bien que l'on rencontre des difficultés pour séparer et récupérer le catalyseur, en particulier quand celui-ci est employé en relativement grande quantité, comme c'est le cas avec les catalyseurs à base de cobalt, ou quand celui-ci est un métal noble, comme c'est le cas avec les catalyseurs à base de rhodium.

Une solution visant à résoudre ce problème a été mentionnée par Bartik et al. : Organometallics (1993) 12 164-170, J. Organometal. Chem. (1994) 480 15-21, ainsi que par Beller et al. : J. Molecular Catal. A : Chemical (1999) 143 31-39. Elle consiste à effectuer l'hydroformylation en présence d'une solution aqueuse contenant un complexe du cobalt qui est rendu hydrosoluble grâce à la présence d'un ligand phosphine-sulfonate tel que le sel de sodium de la triphénylphosphine trisulfonée ou d'une tris-(alkylphényl)-phosphine trisulfonée. La demande de brevet WO-A-97/00132 décrit des clusters de cobalt substitués par des groupements trialkoxysilylméthyle qui les rendent solubles dans l'eau. De cette manière, la phase organique contenant les aldéhydes est aisément séparée de la phase aqueuse contenant le catalyseur.

Une autre solution visant à résoudre ce problème a été décrite dans le brevet français FR-B-2 314 910. Elle consiste à effectuer l'hydroformylation en présence d'une solution aqueuse contenant un complexe du rhodium qui est rendu hydrosoluble grâce à la présence d'un ligand phosphine sulfonée lui-même hydrosoluble tel que le sel de sodium de la triphénylphosphine trisulfonée. De cette manière la phase organique contenant les aldéhydes est aisément séparée de la phase aqueuse contenant le catalyseur. Cette technique a fait l'objet d'un nombre considérable de travaux qui ont été discutés dans un article de W.A. Herrmann paru dans Angewandte Chemie International en 1993, volume 32, page 1524 et suivantes.

En dépit du grand intérêt industriel de ces techniques pour l'hydroformylation du propylène, ces systèmes à deux phases souffrent du manque de solubilité des oléfines dans l'eau, ce qui conduit à des vitesses de réaction relativement faibles qui les rendent inapplicables pour les oléfines à longue chaîne.

Par ailleurs, il a été décrit dans le brevet US-A-3 565 823 une technique consistant à disperser un composé d'un métal de transition dans un sel d'étain ou de germanium et d'un ammonium ou d'un phosphonium quaternaire, de formule (R¹R²R³R⁴Z)YX₃ dans laquelle R¹, R², R³ et R⁴ sont des restes hydrocarbyles ayant jusqu'à 18 atomes de carbone, Z est l'azote ou le phosphore, Y l'étain ou le germanium et X un halogène, par exemple le chlore ou le brome. Dans le brevet US-A-3 832 391 a été décrit un procédé de carbonylation des oléfines par la même composition. Les compositions précédentes présentent le désavantage d'avoir un point de fusion relativement élevé, par exemple supérieur à 90 °C, ce qui complique la manipulation des solutions de catalyseur et des produits de réaction.

Il a été signalé dans le brevet US-A-5 874 638 que l'on peut à la fois bénéficier des avantages d'une mise en oeuvre à deux phases tout en limitant les inconvénients liés d'une part à l'utilisation de l'eau et d'autre part à l'emploi de composés à point de fusion élevé, de la Classification Périodique, en dissolvant certains composés catalytiques des métaux de transition des groupes 8, 9 et 10, connus pour catalyser l'hydroformylation, dans des solvants ioniques non-aqueux qui sont constitués par des sels organiques-inorganiques liquides à température ambiante.

Il a maintenant été trouvé qu'il était possible d'augmenter considérablement les vitesses de réaction en réalisant la réaction dans un liquide ionique partiellement ou complètement miscible avec les produits de la réaction, tout en conservant le bénéfice de la séparation et de la réutilisation du liquide ionique contenant le catalyseur et en améliorant la récupération des produits de la réaction, en injectant après la section réactionnelle un solvant organique (qui peut avantageusement être le composé oléfiniquement insaturé à hydroformyler) peu ou pas miscible avec le liquide ionique et qui améliore la démixtion des produits du reste de l'effluent réactionnel.

Cette mise en oeuvre du procédé permet d'allier les avantages d'un système catalytique homogène tout en apportant une meilleure utilisation du catalyseur et une meilleure récupération des produits.

D'une manière générale, l'invention propose un procédé pour l'hydroformylation en phase liquide d'au moins un composé oléfiniquement insaturé dans lequel on fait réagir dans une section réactionnelle au moins un composé oléfiniquement insaturé avec du monoxyde de carbone et de l'hydrogène, en présence d'au moins un liquide ionique non-aqueux, liquide à la température de la réaction, et au moins partiellement miscible avec les produits de la réaction, le liquide ionique comprenant au moins un sel de formule générale Q⁺ A⁻, dans laquelle Q⁺ représente un ammonium quaternaire et/ou un phosphonium quaternaire, et A⁻ représente un anion, et en présence d'au moins d'un catalyseur comprenant au moins un composé ou un complexe d'un métal de transition des groupes 8, 9 et 10 de la Classification Périodique, éventuellement coordiné par au moins un ligand contenant au moins un atome de phosphore (ligand phosphoré), d'arsenic, d'antimoine ou d'azote (ligand azoté), ledit procédé étant caractérisé en ce que, après la section réactionnelle, on ajoute au milieu au moins un solvant organique, de manière à améliorer la récupération des produits de la réaction.

Plus particulièrement, le procédé de l'invention peut être mis en oeuvre selon un schéma comportant au moins les trois sections suivantes :
a) au moins une section 1, dans laquelle on effectue la réaction d'hydroformylation comme indiqué ci-dessus.
b) au moins une section 2, dans laquelle on effectue
   - le mélange de l'effluent de la section de réaction avec au moins un solvant organique destiné à améliorer la récupération des produits ;
   - la séparation des produits de la réaction et de la phase polaire contenant le liquide ionique et le catalyseur ;
   - et la décantation de la phase polaire contenant le liquide ionique et le catalyseur, qui est recyclée au réacteur de la section 1 pour être réutilisée ;
c) au moins une section 3, dans laquelle on sépare par distillation les produits de la réaction, le solvant organique, qui est recyclé dans la zone de mélange de la section 2, et le composé oléfiniquement insaturé à hydroformyler qui n'a pas réagi, qui est recyclé vers la section 1.

Dans la section 3, suivant que le solvant organique utilisé a un point d'ébullition supérieur ou inférieur à celui du composé oléfiniquement insaturé à hydroformyler, on effectue d'abord la séparation des produits bruts de la réaction et du composé oléfiniquement insaturé à hydroformyler, puis la séparation des produits finaux de la réaction et du solvant organique, ou d'abord la séparation des produits bruts de la réaction et du solvant organique, puis la séparation des produits finaux de la réaction du composé oléfiniquement insaturé à hydroformyler.

Dans une variante préférée du procédé de l'invention, ledit solvant organique utilisé pour améliorer la séparation des produits de la réaction est constitué par le composé oléfiniquement insaturé à hydroformyler qui n'a pas réagi. Il est alors séparé par distillation dans la section 3 et au moins en partie recirculé vers l'opération de mélange de la section 2 et en partie recyclé vers la section de réaction 1. Un avantage particulier de ce mode de réalisation du procédé de l'invention est de ne nécessiter la mise en oeuvre que d'une opération de distillation.

Les composés oléfiniquement insaturés susceptibles d'être hydroformylés sont choisis dans le groupe formé par les monooléfines, les dioléfines et en particulier les dioléfines conjuguées, les composés oléfiniques comportant un ou plusieurs hétéroatomes, notamment dans des groupes insaturés, comme la fonction cétone ou acide carboxylique. A titre d'exemples on peut citer l'hydroformylation des pentènes en hexanal et méthylpentanal, des hexènes en isoheptanals, des isooctènes en isononanals, ou des coupes oléfiniques de C₁₀ à C₁₆ en aldéhydes de C₁₁ à C₁₇.

Le solvant organique est choisi de façon à améliorer la démixtion des produits. Il est choisi plus particulièrement parmi les hydrocarbures aliphatiques, cycliques ou acycliques, saturés ou insaturés, et les hydrocarbures aromatiques ou aromatiques substitués. Parmi ceux-la, on peut choisir de préférence le solvant organique parmi les normales paraffines et isoparaffines et les hydrocarbures aliphatiques cycliques. De la façon la plus préférée, le solvant organique pourra consister en le ou les composés oléfiniquement insaturés à hydroformyler.

Dans le solvant ionique non-aqueux de formule générale Q⁺ A⁻, l'anion A⁻ est choisi de telle manière que le sel Q⁺ A⁻ soit liquide à basse température, c'est-à-dire au-dessous de 90 °C.

Ainsi, les anions A⁻ préférés seront les ions nitrate, sulfate, phosphate, acétate, halogénoacétates, tétrafluoroborate, tétraalkylborates, tétraarylborates, hexafluorophosphate, hexafluoroantimonate, fluorosulfonate, perfluoroalkyl-sulfonates, arène-sulfonates, ces derniers étant éventuellement substitués par des groupements halogènes ou halogénoalkyles, ainsi que les bis-perfluoroalkylsulfonyl amides.

Les cations ammonium et/ou phosphonium quaternaires Q⁺ répondent notamment aux formules générales NR¹R²R³R⁴⁺ et PR¹R²R³R⁴⁺ ou aux formules générales R¹R²N=CR³R⁴⁺ et R¹R²P=CR³R⁴⁺ où R¹, R², R3 et R⁴, identiques ou différents, représentent l'hydrogène (à l'exception du cation NH₄⁺ pour NR¹R²R³R⁴⁺), de préférence un seul substituant représentant l'hydrogène, ou des restes hydrocarbyles ayant de 1 à 30 atomes de carbone, par exemple des groupements alkyles, saturés ou non saturés, cycloalkyles ou aromatiques, aryles ou aralkyles, éventuellement substitués.

Les cations ammonium et/ou phosphonium quaternaires peuvent également être dérivés d'hétérocycles azotés et/ou phosphorés comportant 1, 2 ou 3 atomes d'azote et/ou de phosphore, dans lesquels les cycles sont constitués de 4 à 10 atomes, de préférence de 5 à 6 atomes.

Les cations ammonium et/ou phosphonium quaternaires peuvent également répondre à l'une des formules :

R¹R²⁺N=CR³-R⁵-R³C=N⁺R¹R² et R¹R²⁺P=CR³-R⁵-R³C=P⁺R¹R²

dans lesquelles R¹, R² et R³, identiques ou différents, sont définis comme précédemment et R⁵ représente un reste alkylène ou phénylène.

Parmi les groupements R¹, R², R³ et R⁴, on mentionnera les radicaux méthyle, éthyle, propyle, isopropyle, butyle, secondaire butyle, tertiaire butyle, amyle, phényle ou benzyle ; R⁵ pourra être un groupement méthylène, éthylène, propylène ou phénylène.

Les cations ammonium et/ou phosphonium quaternaires Q⁺ sont choisis de préférence dans le groupe formé par le N-butylpyridinium, le N-éthylpyridinium, le pyridinium, l'éthyl-3 méthyl-1 imidazolium, le butyl-3 méthyl-1 imidazolium, l'hexyl-1 méthyl-3 imidazolium, le diéthylpyrazolium, le N-butyl-N-méthylpyrrolidinium, le triméthylphénylammonium, le tétrabutylphosphonium et le tributyl-(tétradécyl)-phosphonium.

A titre d'exemples des sels utilisables selon l'invention on peut citer l'hexafluorophosphate de N-butylpyridinium, le tétrafluoroborate de N-éthyl-pyridinium, le fluorosulfonate de pyridinium, le tétrafluoroborate de butyl-3 méthyl-1 imidazolium, l'hexafluoroantimonate de butyl-3 méthyl-1 imidazolium, l'hexafluorophosphate de butyl-3 méthyl-1 imidazolium, le trifluoroacétate de butyl-3 méthyl-1 imidazolium, le trifluorométhylsulfonate de butyl-3 méthyl-1 imidazolium, l'hexafluorophosphate de triméthyl-phénylammonium, le trifluoroacétate d'hexyl-1 méthyl-3 imidazolium et le bis[(trifluorométhane) sulfonyl] amidure de butyl-3 méthyl-1 imidazolium. Ces sels peuvent être utilisés seuls ou en mélange.

Les composés des métaux de transition utilisables selon l'invention sont de façon générale tous les composés des métaux de transition des groupes 8, 9 et 10 et notamment ceux connus de l'homme de l'art pour hydroformyler les oléfines. Ils peuvent être complexés ou associés à un ligand organique. Ils peuvent être utilisés seuls ou en mélange. Ils peuvent aussi être mis en oeuvre sous forme de sels qui peuvent être totalement inorganiques, par exemple, mais de façon non préférée, sous la forme d'halogénures. Il s'agit entre autres des composés du cobalt, du rhodium, de l'iridium, du ruthénium, du palladium et du platine.

On peut avantageusement envisager d'associer à tous ces composés des ligands organiques. Ces ligands organiques peuvent être des ligands portant un atome de phosphore (ligands phosphorés), d'arsenic (par exemple des arsines) ou d'antimoine (par exemple des stibines). Ce peuvent aussi être des ligands azotés.

Le ligand azoté est choisi dans le groupe formé par les monoamines, les di-, tri- et polyamines, les imines, les diimines, les pyridines, les bipyridines, les imidazoles, les pyrroles et les pyrazoles.

On peut citer à titre d'exemples non limitatifs la triéthylamine, l'éthylènediamine, la tétraméthyl-éthylènediamine, la diéthylènetriamine, le diazabicyclooctane, le 1,4,7-triméthyl-1,4,7-triazacyclononane, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-bis-(di-t-butyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, la N,N'-bis-(di-t-butyl-2,6-phényl)-butane-2,3-diimine, la pyridine, les picolines, la t-butylpyridine, la bipyridine, la di-t-butyl-bipyridine, l'imidazole, le N-méthylimidazole, le N-butylimidazole, le benzimidazole, le pyrrole, le N-méthylpyrrole, et le 2,6-diméthylpyrrole.

Le ligand azoté peut comporter aussi d'autres fonctions organiques, comme par exemple des fonctions alcool, aldéhyde, cétone, acide, ester ou nitrile. On peut citer à titre d'exemples non limitatifs les acides et esters picoliniques, les 2,6-dialkoxypyridines, les salicylaldimines, les 2,6-bis-N-aryliminopyridines, les 1-dialkyl- et 1-diaryl- phosphino-2-(4-pyridyl)-éthanes, les (pyridyl-4)-2-acétates d'alkyle, les (pyridyl-2)-2-acétates d'alkyle, le bis-(pyridyl-4)-3-propanoate d'éthylèneglycol, le (pyridyl-2)-2-éthanol, le (pyridyl-2)-3-propanol et le (pyridyl-2)-3-propyl acétate.

Le ligand phosphoré est choisi dans le groupe formé par les phosphines tertiaires, les poly-phosphines, les oxydes de phosphines, les phosphites, les chélates phosphine-phosphite, ou phosphine-oxyde de phosphine, ou oxyde de phosphine-phosphite. On peut citer à titre d'exemples non limitatifs les ligands suivants : la triphénylphosphine, la triphénylphosphite et l'oxyde de triphénylphosphine.

Ces ligands peuvent porter en outre, sur l'hétéroatome et/ou la chaîne hydrocarbonée, au moins une autre fonction telle que amine, ammonium, alcool, acide carboxylique, sulfonate, phosphonate, ether, phosphonium, sulfonium. On peut citer à titre d'exemples non limitatifs les ligands suivants : le ligand 1-(4-pyridyl)-2-(dicyclopentylméthylphosphonium)-éthane tétrafluoroborate (**1**), le ligand 1-(N-imidazolyl)-2-(dicyclopentyl-méthyl-phosphonium)-éthane tétrafluoroborate (**2**) le ligand 1-(diphénylphosphino)-2-(4-N-méthyl-pyridinium)-éthane hexafluorophosphate (**3**), le ligand 1-(dicyclopentylphosphino)-2-(3- méthyl-1 imidazolium)-éthane hexafluorophosphate (**4**)

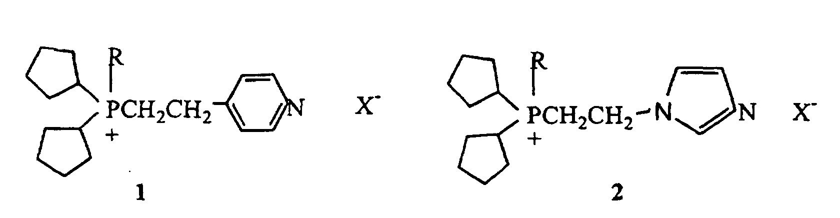

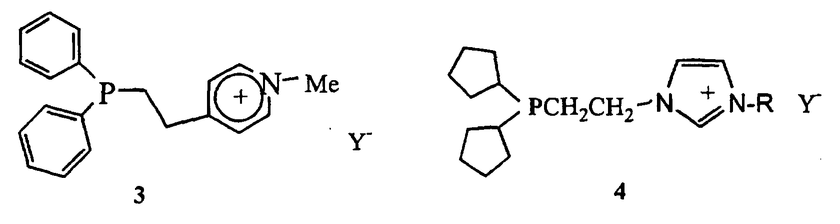

R=Me, X=BF₄, Y=PF₆

le ligand N-(3-diphénylphosphinophényl)-N'-diméthyl-guanidinium tétrafluoroborate (**5**), le ligand tris-(phényl-3-sulfonate de tétrabutylammonium)-phosphine (triphénylphosphine trisulfonate de tétrabutylammonium (**6**) le ligand tris-(phényl-3-sulfonate de sodium)-phosphine (ou triphénylphosphine trisulfonate de sodium ou TPPTS) (**7**), le ligand triphénylphosphine monosulfonate de sodium (ou TPPMS), le ligand (di-t-butyl-3,5-catecholato)-(phénoxy-4-sulfonate de tétrabutylammonium)-phosphite (**8**)

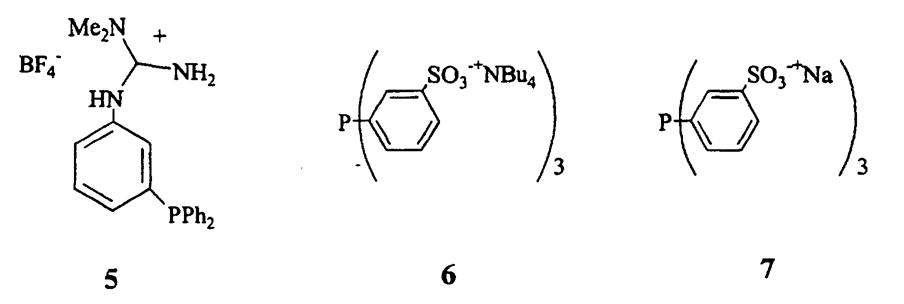

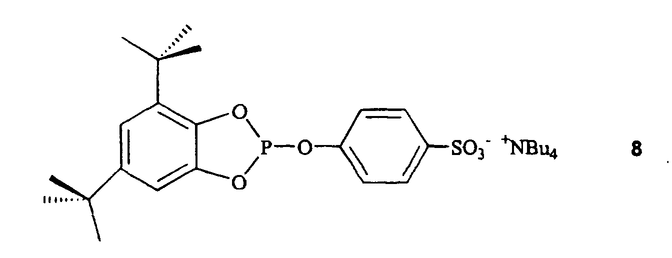

La composition catalytique est obtenue par mélange, d'une manière quelconque, du sel liquide avec le composé du métal de transition et éventuellement le ligand.

Les composants entrant dans la composition selon l'invention peuvent être mélangés dans un ordre quelconque, à une température comprise entre -20 °C et 200 °C, et de préférence entre 0 °C et 140 °C et avantageusement entre 20 °C et 90 °C.

Le complexe peut être préparé *in situ* par mélange du précurseur du métal de transition et du ligand directement dans le réacteur d'hydroformylation.

La concentration du composé de métal de transition dans le solvant ionique liquide n'est pas critique. Elle est avantageusement comprise entre 0,1 mmole par litre de solvant ionique liquide et 5 moles par litre, de préférence entre 1 mmole et 1 mole par litre, et encore entre 10 et 500 mmoles par litre. Le rapport molaire entre le ligand et le composé du métal de transition est compris entre 0,1 et 500, de préférence entre 1 et 100.

Le rapport des pressions partielles de l'hydrogène au monoxyde de carbone utilisé dans le milieu réactionnel pour l'hydroformylation peut être de 10:1 à 1:10, de préférence dans un rapport 1:1, mais tout autre rapport peut être utilisé selon la mise en oeuvre du procédé.

La température à laquelle se fera l'hydroformylation sera comprise entre 30 °C et 200 °C ; avantageusement, elle est inférieure à 150 °C, de préférence entre 50 °C et moins de 150 °C. La pression peut être comprise entre 1 MPa et 20 MPa, de préférence entre 2 MPa et 15 MPa.

Avantageusement, la température et/ou la pression lors de l'addition du solvant sont choisies telles qu'elles sont inférieures ou égales à celles utilisées dans la section réactionnelle.

L'invention est mise en oeuvre dans une installation comportant :
- au moins un réacteur A1 ;
- au moins un mélangeur A2 pour le mélange de l'effluent du réacteur A1 avec au moins un solvant organique ;
- et au moins un décanteur B2 pour la décantation de la phase polaire contenant au moins le liquide ionique contenant au moins le catalyseur qui est recyclé au réacteur A1 ;
ainsi que
- au moins une conduite 1 pour l'introduction de la charge à hydroformyler et du mélange monoxyde de carbone/hydrogène ;
- au moins une conduite 5 pour l'introduction de l'effluent du réacteur dans le mélangeur A2 ;
- au moins une conduite 9 pour l'introduction du solvant organique dans le mélangeur A2 ;
- au moins une conduite 6 pour envoyer vers le décanteur B2 le mélange de l'effluent et du solvant organique contenu dans le mélangeur A2 ; et
- au moins une conduite 7 permettant de renvoyer dans le réacteur A1 la phase polaire contenant au moins le liquide ionique et le catalyseur séparée dans B2 ; et
- au moins une conduite 8 permettant de soutire du décanteur B2 les produits bruts de la réaction.

Dans le cas où le solvant organique utilisé a un point d'ébullition supérieur à celui du composé oléfiniquement insaturé à hydroformyler, l'installation de l'invention comprend en outre :
- dans la section de séparation 3, au moins une colonne A3 pour la séparation des produits bruts de la réaction et du composé oléfiniquement insaturé à hydroformyler qui n'a pas réagi et au moins une colonne B3 permettant de séparer le solvant organique des produits finaux de la réaction ;
ainsi que
- au moins une conduite 4 pour recycler vers le réacteur A1 le composé oléfiniquement insaturé à hydroformyler qui n'a pas réagi séparé dans la colonne A3 ;
- au moins une conduite 10 permettant d'envoyer les produits sortant en pied de la colonne A3 dans la colonne B3 ; et
- au moins une conduite 11 permettant de récupérer les produits finaux de la réaction.

Dans le cas où le solvant organique utilisé a un point d'ébullition inférieur à celui du composé oléfiniquement insaturé à hydroformyler, l'installation de l'invention comprend en outre :
- dans la section de séparation 3, au moins une colonne B3 permettant de séparer le solvant organique des produits bruts de la réaction et au moins une colonne A3 pour la séparation des produits finaux de la réaction et du composé oléfiniquement insaturé à hydroformyler qui n'a pas réagi ;
ainsi que
- au moins une conduite 4 pour recycler vers le réacteur A1 le composé oléfiniquement insaturé à hydroformyler qui n'a pas réagi séparé dans la colonne A3 ;
- au moins une conduite 10 permettant d'envoyer les produits sortant en pied de la colonne B3 dans la colonne A3 ; et
- au moins une conduite 11 permettant de récupérer les produits finaux de la réaction.

Dans le cas où le solvant organique utilisé consiste en le composé oléfiniquement insaturé à hydroformyler lui-même, l'installation de l'invention comprend en outre :
- dans la section de séparation 3, une colonne A3 permettant de séparer du composé oléfiniquement insaturé à hydroformyler qui n'a pas réagi et les produits finaux de la réaction ;
ainsi que
- sortant de cette colonne A3, une conduite pour évacuer le composé oléfiniquement insaturé à hydroformyler qui n'a pas réagi, ladite conduite se subdivisant en deux conduites, la conduite 9 pour envoyer au moins une fraction dudit composé oléfiniquement insaturé dans le mélangeur A2, à titre de solvant organique et une conduite 4 pour recycler une fraction dudit composé oléfiniquement insaturé vers l'entrée du réacteur A1 ; et
- au moins une conduite 11 permettant de récupérer les produits finaux de la réaction.

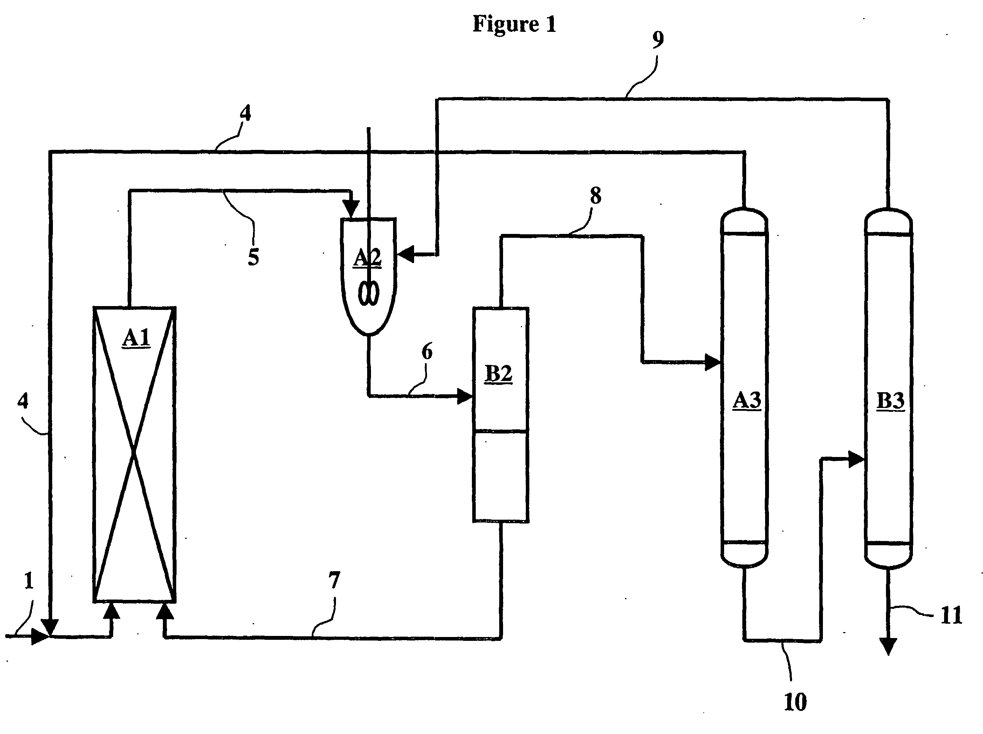

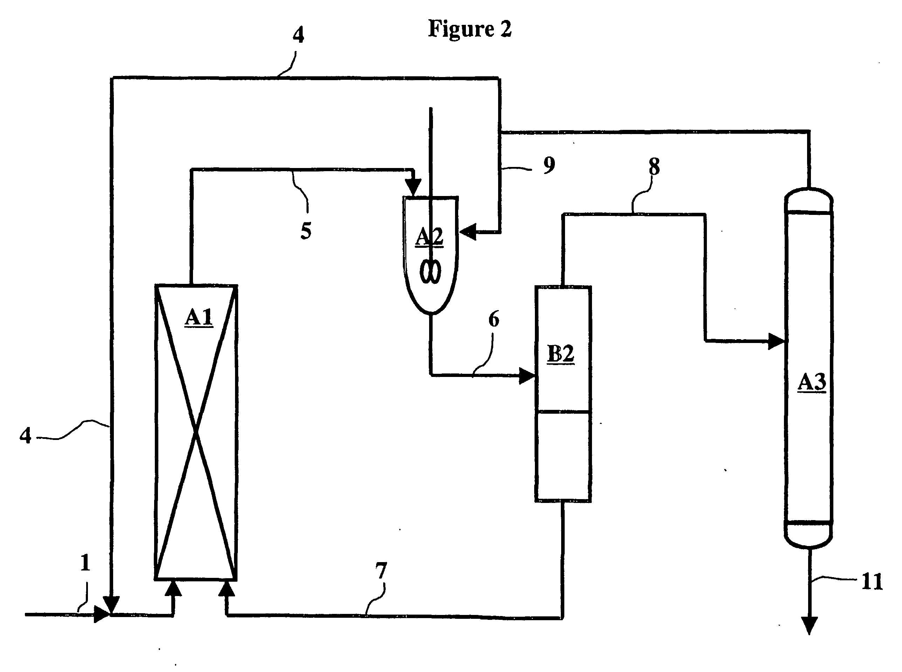

On comprendra mieux le procédé et l'installation de l'invention à partir de la description qui en est faite ci-après, en liaison avec les figures 1 et 2.

La figure 1 illustre un mode de réalisation d'une installation à 3 sections dans lequel le solvant organique choisi a un point d'ébullition plus élevé que le composé à hydroformyler. Selon une autre option, le solvant organique peut aussi avoir un point d'ébullition inférieur à celui des réactifs et des produits.

Selon la figure 1, la réaction est réalisée dans le réacteur A1 en présence de la charge à hydroformyler, qui peut être introduite par la ligne 1, du (ou des) composé(s) de métal de transition, du ligand organique (qui optionnellement peut être introduit en mélange avec le ou les composés des métaux de transition), de monoxyde de carbone et d'hydrogène, qui peuvent être introduits par la ligne 1, et en présence d'au moins un liquide ionique, mis en oeuvre en phase liquide, en milieu homogène ou biphasique. Le liquide ionique peut être introduit dans le réacteur au début de la réaction. Optionnellement, du liquide ionique frais peut être injecté dans le réacteur A1 au cours de la réaction et du liquide ionique usé soutiré de A1.

La chaleur de réaction est éliminée par les techniques connues de l'homme du métier qui ne sont pas représentées sur la figure 1.

A la sortie de la section réactionnelle, l'effluent du réacteur est envoyé, par la ligne 5, dans au moins un mélangeur A2 dans lequel est injecté, par la ligne 9, un solvant organique qui améliore la démixtion des produits de la réaction. L'effluent est ensuite soutiré du mélangeur A2 et envoyé dans un décanteur B2 par la ligne 6. Dans ce décanteur B2, la phase polaire qui contient au moins le liquide ionique et le catalyseur est séparée du mélange des produits et du solvant organique et est renvoyé au réacteur A1 par la ligne 7. La phase polaire ainsi renvoyé au réacteur peut optionnellement contenir une fraction du solvant organique.

Les produits de la réaction en mélange avec le solvant organique sont envoyés dans une première colonne de distillation A3 par la ligne 8. Dans la colonne A3 on sépare en tête le composé oléfiniquement insaturé à hydroformyler qui n'a pas réagi ; il est recyclé au réacteur A1 par la ligne 4. Les produits et le solvant sont envoyés dans une colonne B3 par la ligne 10. Dans cette colonne B3, et dans un mode de réalisation, le solvant organique est séparé en tête de colonne, dans l'option où il est plus léger que les produits, et envoyé dans le mélangeur A2 par la ligne 9. En pied de colonne B3, les produits sont récupérés par la ligne 11. Ils peuvent alors, si nécessaire, être envoyés dans une zone de fractionnement ultérieur.

La colonne A3 est installée avant la colonne B3 ou l'inverse, selon que le point d'ébullition du solvant organique est supérieur ou inférieur à celui du composé à hydroformyler.

La figure 2 illustre la variante préférée du procédé de l'invention, selon laquelle le solvant organique consiste en le composé oléfiniquement insaturé à hydroformyler. Cette option présente l'avantage de mettre en oeuvre une colonne de distillation en moins dans la section 3.

Selon la figure 2, la réaction est réalisée dans le réacteur A1 en présence de la charge à hydroformyler, qui peut être introduite par la ligne 1, du ou des composé(s) de métal de transition, du ligand organique (qui peut être introduit en mélange avec le ou les composés des métaux de transition), de monoxyde de carbone et d'hydrogène, qui peuvent être introduits par la ligne 1 et en présence d'au moins un liquide ionique, mis en oeuvre en phase liquide, en milieu homogène ou biphasique. Le liquide ionique peut être introduit dans le réacteur au début de la réaction. Du liquide ionique frais peut éventuellement être injecté dans le réacteur A1 au cours de la réaction et du liquide ionique usé soutiré du réacteur A1.

La chaleur de réaction est éliminée par les techniques connues de l'homme du métier qui ne sont pas représentées sur la figure 2.

A la sortie de la section réactionnelle, l'effluent du réacteur est envoyé, par la ligne 5, dans au moins un mélangeur A2 dans lequel est injecté, par la ligne 9, un solvant organique qui améliore la démixtion des produits de la réaction. Avantageusement, dans l'option décrite par la figure 2, ce solvant organique est constitué par le ou les réactifs.

L'effluent est ensuite soutiré du mélangeur A2 et envoyé dans un décanteur B2 par la ligne 6. Dans ce décanteur B2, la phase polaire qui contient au moins le liquide ionique et le catalyseur est séparée du mélange des produits et du solvant organique et est renvoyé au réacteur A1 par la ligne 7.

Les produits de la réaction et le solvant sont envoyés, par la ligne 8, dans la colonne à distiller A3. Dans une option de réalisation, les réactifs sont séparés en tête de colonne et envoyés par la ligne 9 dans le mélangeur A2. En pied de colonne A3, les produits sont récupérés par la ligne 11. Ils peuvent alors, si nécessaire, être envoyés dans une zone de fractionnement ultérieur.

Dans l'option illustrée par la figure 2, le débit de recirculation du composé à hydroformyler (ligne 9) est ajusté de façon à ce que la majeure partie des produits démixent dans le décanteur B2.

Les exemples suivants illustrent l'invention sans en limiter la portée.

### EXEMPLE 1

La réaction d'hydroformylation de l'hexène-1 est conduite dans un autoclave en acier inoxydable d'une contenance de 100 mL, muni d'une double enveloppe permettant la régulation de la température par circulation d'un fluide caloporteur. Dans cet autoclave purgé au préalable de l'air et de l'humidité et placé sous pression atmosphérique du mélange hydrogène-monoxyde de carbone (1/1 molaire), on introduit 0,0193 g d'acétylacétonate de rhodium-dicarbonyle (soit 0,075 mmole de rhodium), 4 équivalents molaires de triphénylphosphine-trisulfonate de sodium, 4 mL de liquide ionique trifluoroacétate de butyl-3 méthyl-1 imidazolium et 7,5 mL d'hexène-1. On porte la pression du mélange hydrogène-monoxyde de carbone (1/1 molaire) à 2 MPa et la température à 80 °C et on met l'agitation en route. Après 1 heure, on arrête l'agitation et on laisse refroidir le mélange réactionnel, puis on relâche la pression. L'effluent du réacteur est soutiré. La majorité de la phase organique est miscible dans le liquide ionique. On ajoute 8 mL d'heptane qui entraînent une démixtion des produits.

L'analyse de la phase organique est réalisée par chromatographie en phase gazeuse. La conversion de l'hexène est de 97 %. La sélectivité en aldéhydes est de 98 % et le rapport n/i (n-heptanal / isoheptanals) de 3,1. La fréquence de rotation est calculée à 25 % de conversion. Elle est égale à 33 moles d'aldéhydes formées par mole de rhodium et par heure.

### EXEMPLE 2

La réaction d'hydroformylation de l'hexène-1 est conduite dans le même appareillage et selon le même mode opératoire que décrit dans l'exemple 1 à l'exception que l'on change la nature du cation constituant le liquide ionique. Dans cet autoclave purgé au préalable de l'air et de l'humidité et placé sous pression atmosphérique du mélange hydrogène-monoxyde de carbone (1/1 molaire), on introduit 0,0193 g d'acétylacétonate de rhodium-dicarbonyle (soit 0,075 mmole de rhodium), 4 équivalents molaire de triphénylphosphine-trisulfonate de sodium, 4 mL de liquide ionique trifluoroacétate d'hexyl-1 méthyl-3 imidazolium et 7,5 mL d'hexène-1. On porte la pression du mélange hydrogène-monoxyde de carbone (1/1 molaire) à 2 MPa et la température à 80 °C et on met l'agitation en route. Après 1 heure, on arrête l'agitation et on laisse refroidir le mélange réactionnel, puis on relâche la pression. L'effluent du réacteur est soutiré. L'effluent du réacteur est constitué par une seule phase liquide. On ajoute 8 mL d'heptane qui entraînent une démixtion des produits.

L'analyse de la phase organique est réalisée par chromatographie en phase gazeuse. La conversion de l'hexène est totale. La sélectivité en aldéhydes est de 99 % et le rapport n/i (n-heptanal / isoheptanals) est de 2,9. La fréquence de rotation est calculée à 25 % de conversion. Elle est égale à 60 moles d'aldéhydes formées par mole de rhodium et par heure. La vitesse initiale de la réaction est donc considérablement améliorée par la mise en oeuvre d'un liquide ionique qui présente une affinité plus grande pour les aldéhydes.

### EXEMPLE 3 (récupération du rhodium dans le liquide ionique)

La réaction d'hydroformylation de l'hexène-1 est conduite comme dans les Exemples 1 et 2. Dans l'autoclave purgé au préalable de l'air et de l'humidité et placé sous pression atmosphérique du mélange hydrogène-monoxyde de carbone (1/1 molaire), on introduit 0,0193 g d'acétylacétonate de rhodium-dicarbonyle (soit 0,075 mmole de rhodium), 4 équivalents molaires de triphénylphosphine-trisulfonate de sodium, 4 mL de bis[(trifluorométhane)sulfonyl] amidure de butyl-3 méthyl-1 imidazolium et 7,5 mL d'hexène-1. On porte la pression du mélange hydrogène-monoxyde de carbone (1/1 molaire) à 2 MPa et la température à 80 °C et on met l'agitation en route. Après 1 heure, on arrête l'agitation et on laisse refroidir le mélange réactionnel, puis on relâche la pression. Après soutirage hors de l'autoclave, le mélange réactionnel est composé d'une seule phase liquide. On ajoute 8 mL d'heptane qui engendrent une démixtion des produits dans une 2^{ème} phase. La conversion de l'hexène-1 est de 99 % en poids. La sélectivité pour les aldéhydes en C7 est de 93 % et le rapport n/iso (n-heptanal / isoheptanals) est égal à 3,5. L'analyse de la phase organique supérieure montre qu'elle contient moins de 5 ppm de rhodium métal (ppm : parties par million en poids), ce qui correspond à moins de 0,2 % en poids du métal engagé.

La phase supérieure organique obtenue, qui contient l'heptane et les produits de la réaction, est distillée de façon à séparer l'heptane.

La phase ionique inférieure est réinjectée dans l'autoclave purgé au préalable de l'air et de l'humidité et placé sous pression atmosphérique du mélange hydrogène-monoxyde de carbone (1/1 molaire). On introduit alors 7,5 mL d'hexène-1. On porte la pression du mélange hydrogène-monoxyde de carbone (1/1 molaire) à 2 MPa et la température à 80 °C et on met l'agitation en route. Après 1 heure, on arrête l'agitation et on laisse refroidir le mélange réactionnel, puis on relâche la pression. Après soutirage hors de l'autoclave, le mélange réactionnel est composé d'une seule phase liquide. On ajoute alors l'heptane récupéré par distillation du premier batch de l'Exemple 3 ce qui engendre une démixtion des produits dans une 2^{ème} phase. La conversion de l'hexène-1 est de 98 % en poids. La sélectivité pour les aldéhydes en C7 est de 93 % et le rapport n/iso (n-heptanal / isoheptanals) est égal à 3,5.

### EXEMPLE 4

La réaction d'hydroformylation est conduite comme dans l'Exemple 3. Dans l'autoclave purgé au préalable de l'air et de l'humidité et placé sous pression atmosphérique du mélange hydrogène-monoxyde de carbone (1/1 molaire), on introduit 0,0193 g d'acétylacétonate de rhodium-dicarbonyle (soit 0,075 mmole de rhodium), 4 équivalents molaires de triphénylphosphine-trisulfonate de sodium, 4 mL de bis[(trifluorométhane)sulfonyl] amidure de butyl-3 méthyl-1 imidazolium et 7,5 mL d'hexène-1. On porte la pression du mélange hydrogène-monoxyde de carbone (1/1 molaire) à 2 MPa et la température à 80 °C et on met l'agitation en route. Après 1 heure, on arrête l'agitation et on laisse refroidir le mélange réactionnel, puis on relâche la pression. Après soutirage hors de l'autoclave, le mélange réactionnel est composé d'une seule phase liquide. On ajoute alors 8 mL d'hexène-1 qui engendrent, de la même façon qu'avec l'heptane décrit dans l'Exemple 3, une démixtion des produits dans une 2^{ème} phase. La conversion de l'hexène-1, calculée par rapport au 7,5 mL d'hexène-1 introduit au début de la réaction, est de 99 % en poids. La sélectivité pour les aldéhydes en C7 est de 93 % et le rapport n/iso (n-heptanal / isoheptanals) est égal à 3,5. L'analyse de la phase organique supérieure montre qu'elle contient moins de 5 ppm de rhodium métal (ppm : parties par million en poids), ce qui correspond à moins de 0,2 % en poids du métal engagé.

## Revendications

1. Procédé pour l'hydroformylation en phase liquide d'au moins un composé oléfiniquement insaturé dans lequel on fait réagir dans une section réactionnelle au moins un composé oléfiniquement insaturé avec du monoxyde de carbone et de l'hydrogène, en présence d'au moins une phase polaire contenant
- un liquide ionique non-aqueux, liquide à la température de la réaction, et au moins partiellement miscible avec les produits de la réaction, le liquide ionique comprenant au moins un sel de formule générale Q⁺ A⁻, dans laquelle Q⁺ représente un ammonium quaternaire et/ou un phosphonium quaternaire, et A⁻ représente un anion ;
- et au moins d'un catalyseur comprenant au moins un composé ou un complexe d'un métal de transition des groupes 8, 9 et 10 de la Classification Périodique, éventuellement coordiné par au moins un ligand contenant au moins un atome de phosphore, d'arsenic, d'antimoine ou d'azote,
ledit procédé étant **caractérisé en ce que**, après la section réactionnelle, on ajoute au milieu au moins un solvant organique, de manière à améliorer la séparation par démixtion des produits bruts de la réaction de ladite phase polaire, qui est recyclée à la section réactionnelle pour être réutilisée.

2. Procédé selon la revendication 1 **caractérisé en ce qu'**il comporte au moins les trois sections suivantes :
a) au moins une section 1, dans laquelle on effectue la réaction d'hydroformylation.
b) au moins une section 2, dans laquelle on effectue
- le mélange de l'effluent de la section de réaction avec au moins un solvant organique destiné à améliorer la récupération des produits ;
- la séparation des produits de la réaction et de la phase polaire contenant le liquide ionique et le catalyseur ;
- et la décantation de la phase polaire contenant le liquide ionique et le catalyseur ;
c) au moins une section 3, dans laquelle on sépare par distillation les produits de la réaction, le solvant organique, qui est recyclé dans la zone de mélange de la section 2, et le composé oléfiniquement insaturé à hydroformyler qui n'a pas réagi, qui est recyclés vers la section 1.

3. Procédé selon la revendication 2 **caractérisé en ce que** le solvant organique utilisé ayant un point d'ébullition supérieur à celui du composé oléfiniquement insaturé à hydroformyler, on effectue d'abord la séparation des produits bruts de la réaction et du composé oléfiniquement insaturé à hydroformyler, puis la séparation des produits finaux de la réaction et du solvant organique.

4. Procédé selon la revendication 2 **caractérisé en ce que** le solvant organique utilisé ayant un point d'ébullition inférieur à celui du composé oléfiniquement insaturé à hydroformyler, on effectue d'abord la séparation des produits bruts de la réaction et du solvant organique, puis la séparation des produits finaux de la réaction du composé oléfiniquement insaturé à hydroformyler.

5. Procédé selon la revendication 1 à 4 **caractérisé en ce que** ledit composé oléfiniquement insaturé à hydroformyler est choisi dans le groupe formé par les monooléfines, les dioléfines, les dioléfines conjuguées, les composés oléfiniques comportant un ou plusieurs hétéroatomes, notamment dans des groupements insaturés.

6. Procédé selon l'une des revendications 1 à 5 **caractérisé en ce que** le solvant organique est choisi parmi les hydrocarbures aliphatiques cycliques ou acycliques saturés ou insaturés, aromatiques ou aromatiques substitués.

7. Procédé selon l'une des revendications 1 à 6 **caractérisé en ce que** le solvant organique est le composé oléfiniquement insaturé à hydroformyler lui-même.

8. Procédé selon l'une des revendications 1 à 7 **caractérisé en ce que**, dans le sel de formule générale Q⁺ A⁻, l'anion A⁻ est choisi de telle manière que le sel Q⁺ A⁻ soit liquide au-dessous de 90 °C.

9. Procédé selon l'une des revendications 1 à 8 **caractérisé en ce que** l'anion A⁻ est choisi est choisi dans le groupe formé par les ions nitrate, sulfate, phosphate, acétate, halogénoacétates, tétrafluoroborate, tétraalkylborates, tétraarylborates, tétrachloroborate, hexafluorophosphate, hexafluoroantimonate, fluorosulfonate, perfluoroalkylsulfonates, arène-sulfonates, arène-sulfonates substitués par des groupements halogènes ou halogénoalkyles et bis-perfluoroalkylsulfonyl amides.

10. Procédé selon l'une des revendications 1 à 9 **caractérisé en ce que** le cation ammonium et/ou phosphonium quaternaire répond à l'une des formules générales :
NR¹R²R³R⁴⁺ et PR¹R²R³R⁴⁺
ou à l'une des formules générales :
R¹R²N=CR³R⁴⁺ et R¹R²P=CR³R⁴⁺,
dans lesquelles R¹, R², R³ et R⁴, identiques ou différents, représentent chacun l'hydrogène, à l'exception du cation NH₄⁺ pour NR¹R²R³R⁴⁺, de préférence un seul substituant représentant l'hydrogène, ou des restes hydrocarbyles ayant de 1 à 30 atomes de carbone.

11. Procédé selon l'une des revendications 1 à 9 **caractérisé en ce que** le cation ammonium et/ou phosphonium quaternaire est dérivé d'un hétérocycle azoté et/ou phosphoré comportant 1, 2 ou 3 atomes d'azote et/ou de phosphore, dans lequel les cycles sont constitués de 4 à 10 atomes.

12. Procédé selon l'une des revendications 1 à 7 **caractérisé en ce que** le cation ammonium et/ou phosphonium quaternaire répond à l'une des formules :
R¹R²⁺N=CR³-R⁵-R³C=N⁺R¹R² et R¹R²⁺P=CR³-R⁵-R³C=P⁺R¹R²
dans lesquelles R¹, R² et R³, identiques ou différents, sont définis comme dans la revendication 4 et R⁵ représente un reste alkylène ou phénylène.

13. Procédé selon l'une des revendications 1 à 7 **caractérisé en ce que** le cation ammonium et/ou phosphonium quaternaire est choisi dans le groupe formé par le N-butylpyridinium, le N-éthylpyridinium, le pyridinium, l'éthyl-3 méthyl-1 imidazolium, le butyl-3 méthyl-1 imidazolium, l'hexyl-1 méthyl-3 imidazolium le diéthylpyrazolium, le N-butyl-N-méthylpyrrolidinium, le triméthylphénylammonium, le tétrabutylphosphonium et le tributyl-(tétradécyl)-phosphonium.

14. Procédé selon l'une des revendications 1 à 13 **caractérisé en ce que** le solvant ionique non-aqueux est au moins un sel choisi dans le groupe formé par l'hexafluorophosphate de N-butyl-pyridinium, le tétrafluoroborate de N-éthylpyridinium, le fluorosulfonate de pyridinium, le tétrafluoroborate de butyl-3-méthyl-1 imidazolium, l'hexafluoroantimonate de butyl-3 méthyl-1 imidazolium, l'hexafluorophosphate de butyl-3 méthyl-1 imidazolium, le trifluoroacétate de butyl-3 méthyl-1 imidazolium, le trifluorométhylsulfonate de butyl-3 méthyl-1 imidazolium, l'hexafluorophosphate de triméthyl-phénylammonium, le trifluoroacétate d'hexyl-1-méthyl-3 imidazolium, le bis[(trifluorométhane) sulfonyl] amidure de butyl-3-méthyl-1-imidazolium.

15. Procédé selon l'une des revendications 1 à 14 **caractérisé en ce que** le métal de transition est le cobalt, le rhodium, l'iridium, le ruthénium, le palladium et le platine.

16. Procédé selon l'une des revendications 1 à 15 **caractérisé en ce que** le composé de métal de transition est un complexe de métal de transition.

17. Procédé selon l'une des revendications 1 à 16 **caractérisé en ce que** la composition catalytique contient également un ligand ou un ligand associé au composé du métal de transition, ledit ligand étant choisi dans le groupe formé par les ligands portant au moins un atome de phosphore (ligands phosphorés), d'arsenic ou d'antimoine ou d'azote (ligands azotés).

18. Procédé selon la revendication 17 **caractérisé en ce que** le ligand est choisi parmi les phosphines tertiaires, les poly-phosphines, les oxydes de phosphines, les phosphites, les chélates phosphine-phosphites, phosphine-oxyde de phosphine et oxyde de phosphine-phosphites, les arsines, les stibines et les ligands azotés.

19. Procédé selon la revendication 18 **caractérisé en ce que** le ligand phosphoré contient au moins une fonction amine, ammonium, alcool, acide carboxylique, sulfonate, phosphonate, éther, phosphonium ou sulfonium.

20. Procédé selon la revendication 17 **caractérisé en ce que** le ligand azoté est choisi dans le groupe formé par les monoamines, les di-, tri- et polyamines, les imines, les diimines, les pyridines, les bipyridines, les imidazoles, les pyrroles et les pyrazoles

21. Procédé selon l'une des revendications 1 à 20 **caractérisé en ce que** la concentration du métal de transition dans le solvant ionique liquide est comprise entre 0,1 mmole par litre et 5 moles par litre et le rapport molaire entre le ligand et le composé du métal de transition est compris entre 0,1 et 500.

22. Procédé selon l'une des revendications 1 à 21 **caractérisé en ce que** la réaction d'hydroformylation est effectuée avec un rapport des pressions partielles de l'hydrogène au monoxyde de carbone de 10:1 à 1:10, à une température comprise entre 30 °C et 200 °C et sous une pression comprise entre 1 MPa et 20 MPa.

23. Procédé selon l'une des revendications 1 à 22 **caractérisé en ce que** la température et/ou la pression lors de l'addition du solvant sont choisies de telle façon telles qu'elles sont inférieures ou égales celles mises en oeuvre dans la section réactionnelle.

## Patentansprüche

1. Verfahren zur Hydroformylierung in flüssiger Phase von wenigstens einer olefinisch ungesättigten Verbindung, in dem man in einem Reaktionsabschnitt wenigstens eine olefinisch ungesättigte Verbindung mit Kohlenmonoxid und Wasserstoff in Gegenwart wenigstens einer polaren Phase reagieren lässt, die enthält:
- eine nicht wässrige ionische Flüssigkeit, die bei der Reaktionstemperatur flüssig ist und wenigstens teilweise mit den Reaktionsprodukten mischbar ist, wobei die ionische Flüssigkeit wenigstens ein Salz mit einer allgemeinen Formel Q⁺A⁻ umfasst, in der Q⁺ ein quarternäres Ammonium und/oder ein quarternäres Phosphonium darstellt und A⁻ ein Anion darstellt;
- und wenigstens einen Katalysator, der wenigstens eine Verbindung oder einen Komplex eines Übergangsmetalls der Gruppen 8, 9 und 10 des Periodensystems, gegebenenfalls koordiniert durch wenigstens einen Liganden umfasst, der wenigstens ein Phosphor-, Arsen-, Antimon- oder Stickstoffatom enthält,
wobei das Verfahren **dadurch gekennzeichnet ist, dass** man nach dem Reaktionsabschnitt dem Medium wenigstens ein organisches Lösungsmittel derart zugibt, dass die Trennung durch Entmischung der Rohprodukte der Reaktion von der polaren Phase verbessert wird, welche zum Reaktionsabschnitt rezykliert wird, um wiederverwendet zu werden.

2. Verfahren nach Anspruch 1, **dadurch gekennzeichnet, dass** es wenigstens die drei folgenden Abschnitte aufweist:
a) wenigstens einen Abschnitt 1, in dem man die Hydroformylierungsreaktion durchführt,
b) wenigstens einen Abschnitt 2, in dem man durchführt:
- das Mischen des Abstroms des Reaktionsabschnitts mit wenigstens einem organischen Lösungsmittel, das dazu vorgesehen ist, die Gewinnung der Produkte zu verbessern;
- die Trennung der Produkte der Reaktion und der polaren Phase, die die ionische Flüssigkeit und den Katalysator enthält;
- und das Dekantieren der polaren Phase die die ionische Flüssigkeit und den Katalysator enthält;
c) wenigstens einen Abschnitt 3, in dem man durch Destillation die Reaktionsprodukte, das organische Lösungsmittel, das in die Mischzone des Abschnitts 2 rezykliert wird, und die zu hydroformylierende olefinisch ungesättigte Verbindung, die nicht reagiert hat trennt, welche zum Abschnitt 1 rezykliert wird.

3. Verfahren nach Anspruch 2, **dadurch gekennzeichnet, dass** das verwendete organische Lösungsmittel einen Siedepunkt über jenem der zu hydroformylierenden olefinisch ungesättigten Verbindung hat, man zuerst die Trennung der Rohprodukte der Reaktion und der zu hydroformylierenden olefinisch ungesättigten Verbindung durchführt und dann die Trennung der Endprodukte der Reaktion und des organischen Lösungsmittels.

4. Verfahren nach Anspruch 2, **dadurch gekennzeichnet, dass** das verwendete organische Lösungsmittel einen Siedepunkt unter jenem der zu hydroformylierenden olefinisch ungesättigten Verbindung hat, man zuerst die Trennung der Rohprodukte der Reaktion und des organischen Lösungsmittels durchführt und dann die Trennung der Endprodukte der Reaktion von der zu hydroformylierenden olefinisch ungesättigten Verbindung.

5. Verfahren nach einem der Ansprüche 1 bis 4, **dadurch gekennzeichnet, dass** die zu hydroformylierende olefinisch ungesättigte Verbindung gewählt wird aus der Gruppe, die gebildet wird durch die Monoolefine, die Diolefine, die konjugierten Diolefine, die olefinischen Verbindungen, die eines oder mehrere Heteroatome aufweisen, insbesondere in den ungesättigten Gruppen.

6. Verfahren nach einem der Ansprüche 1 bis 5, **dadurch gekennzeichnet, dass** das organische Lösungsmittel gewählt wird unter den gesättigten oder ungesättigten aliphatischen, zyklischen oder azyklischen, aromatischen oder substituierten aromatischen Kohlenwasserstoffen.

7. Verfahren nach einem der Ansprüche 1 bis 6, **dadurch gekennzeichnet, dass** das organische Lösungsmittel die zu hydroformylierende olefinisch ungesättigte Verbindung selbst ist.

8. Verfahren nach einem der Ansprüche 1 bis 7, **dadurch gekennzeichnet, dass** in dem Salz mit einer allgemeinen Formel Q⁺A⁻ das Anion A⁻ derart gewählt wird, dass das Salz Q⁺A⁻ unter 90°C flüssig ist.

9. Verfahren nach einem der Ansprüche 1 bis 8, **dadurch gekennzeichnet, dass** das Anion A⁻ gewählt wird aus der Gruppe die gebildet wird durch die Ionen Nitrat, Sulfat, Phosphat, Acetat, Halogenacetate, Tetrafluorborat, Tetraalkylborate, Tetraarylborate, Tetrachlorborat, Hexafluorphosphat, Hexafluorantimonat, Fluorsulfonat, Perfluoralkylsulfonate, Arensulfonate, durch Halogen- oder Halogenalkylgruppen substituierte Arensulfonate und Bis-Perfluoralkylsulfonylamide.

10. Verfahren nach einem der Ansprüche 1 bis 9, **dadurch gekennzeichnet, dass** das quarternäre Ammonium- und oder Phosphoniumkation einer der allgemeinen Formeln:
NR¹R²R³R⁴⁺ und PR¹R ²R ³R⁴⁺
entspricht oder einer der allgemeinen Formeln:
R¹R²N=CR³R⁴⁺ und R¹R²P=CR³R⁴,
in denen R¹, R², R³ und R⁴⁺ identisch oder verschieden jeder Wasserstoff, mit Ausnahme des NH₄⁺-Kations für NR¹R²R³R⁴⁺, wobei vorzugsweise ein einziger Substituent Wasserstoff darstellt, oder Kohlenwasserstoffreste mit 1 bis 30 Kohlenstoffatomen darstellen.

11. Verfahren nach einem der Ansprüche 1 bis 9, **dadurch gekennzeichnet, dass** das quartemäre Ammonium- und oder Phosphoniumkation von einem stickstoff- und/oder phosphorhaltigem Heterozyklus abgeleitet ist, der 1, 2 oder 3 Stickstoff- und/oder Phosphoratome umfasst, in dem die Zyklen aus 4 bis 10 Kohlenstoffatomen bestehen.

12. Verfahren nach einem der Ansprüche 1 bis 7, **dadurch gekennzeichnet, dass** das quarternäre Ammonium- und oder Phosphoniumkation einer der allgemeinen Formeln:
R¹R²⁺N=CR³-R⁵- R³C=N⁺R¹R² und R¹R²⁺P=CR³-R⁵- R³C=P⁺R¹R²
entspricht, in denen R¹, R² und R³ identisch oder verschieden wie nach Anspruch 4 definiert sind und R⁵ einen Alkylen- oder Phenylenrest darstellt.

13. Verfahren nach einem der Ansprüche 1 bis 7, **dadurch gekennzeichnet, dass** das quarternäre Ammonium- und oder Phosphoniumkation gewählt wird aus der Gruppe, die gebildet wird durch N-Butylpyridinium, N-Ethylpyridinium, Pyridinium, 1-Methyl-3-Ethylimidazolium, 1-Methyl-3-Butylimidazolium, 1-Hexyl-3-Methylimidazolium, Diethylpyrazolium, N-Butyl-N-Methylpyrrolidinium, Trimethylphenylammonium, Tetrabutylphosphonium und Tributyl-(Tetradecyl)-Phosphonium.

14. Verfahren nach einem der Ansprüche 1 bis 13, **dadurch gekennzeichnet, dass** das nicht wässrige ionische Lösungsmittel wenigstens ein Salz ist, das aus der Gruppe gewählt wird, die gebildet wird durch Hexafluorphosphat, von N-Butylpyridinium, Tetrafluorborat von N-Ethylpyridinium, Pyridinium-Fluorsulfonat, Tetrafluorborat von 1-Methyl-3-Butylimidazolium, Hexafluorantimonat von 1-Methyl-3-Butylimidazolium, Hexafluorphosphat von 1-Methyl-3-Butylimidazolium, Trifluoracetat von 1-Methyl-3-Butylimidazolium, Trifluormethylsulfonat von 1-Methyl-3-Butylimidazolium, Hexafluorphosphat von Trimethylphenylamonium, Trifluoracetat von 1-Hexyl-3-Methylimidazolium, Bis[(Trifluormethan)-Sulfonyl]-Amidin von 1-Methyl-3-Butylimidazolium.

15. Verfahren nach einem der Ansprüche 1 bis 14, **dadurch gekennzeichnet, dass** das Übergangsmetall Kobalt, Rhodium, Iridium, Ruthenium, Palladium und Platin ist.

16. Verfahren nach einem der Ansprüche 1 bis 15, **dadurch gekennzeichnet, dass** die Übergangsmetallverbindung ein Komplex eines Übergangsmetalls ist.

17. Verfahren nach einem der Ansprüche 1 bis 16, **dadurch gekennzeichnet, dass** die katalytische Zusammensetzung auch einen Liganden enthält oder einen Liganden, der der Übergangsmetallverbindung zugeordnet ist, wobei der Ligand aus der Gruppe gewählt wird, die gebildet wird durch die Liganden, die wenigstens ein Phosphoratom (phosphorhaltige Liganden), Arsenatom oder Antimonatom oder Stickstoffatom (stickstoffhaltige Liganden) tragen.

18. Verfahren nach Anspruch 17, **dadurch gekennzeichnet, dass** der Ligand gewählt wird unter den tertiären Phosphinen, den Polyphosphinen, den Oxiden von Phosphinen, den Phosphiten, den Chelaten Phosphin-Phosphite, Phosphin-Phosphinoxid und Phosphinoxid-Phosphite, den Arsinen, den Stibinen und den stickstoffhaltigen Liganden.

19. Verfahren nach Anspruch 18, **dadurch gekennzeichnet, dass** der phosphorhaltige Ligand wenigstens eine Amin-, Ammonium-, Alkohol-, Carbonsäure-, Sulfonat-, Phosphonat-, Ether-, Phosphonium-, oder Sulfoniumfunktion enthält.

20. Verfahren nach Anspruch 17, **dadurch gekennzeichnet, dass** der stickstoffhaltige Ligand aus der Gruppe gewählt wird, die gebildet wird durch die Monoamine, die Di-, Tri- und Polyamine, die Imine, die Diimine, die Pyridine, die Bipyridine, die Imidazole, die Pyrrole und die Pyrazole.

21. Verfahren nach einem der Ansprüche 1 bis 20, **dadurch gekennzeichnet, dass** die Übergangsmetallkonzentration in dem ionischen flüssigen Lösungsmittel zwischen 0,1 mmol pro Liter und 5 mol pro Liter liegt und das Molverhältnis zwischen dem Ligand und der Übergangsmetallverbindung zwischen 0,1 und 500 liegt.

22. Verfahren nach einem der Ansprüche 1 bis 21, **dadurch gekennzeichnet, dass** die Hydroformylierungsreaktion mit einem Verhältnis der Partialdrucke von Wasserstoff und Kohlenmonoxid von 10:1 bis 1:10 bei einer Temperatur zwischen 30°C und 200°C und unter einem Druck zwischen 1 MPa und 20 MPa durchgeführt wird.

23. Verfahren nach einem der Ansprüche 1 bis 22, **dadurch gekennzeichnet, dass** die Temperatur und/oder der Druck bei der Zugabe des Lösungsmittels derart gewählt werden, das sie kleiner oder gleich jenen sind, die indem Reaktionsabschnitt eingesetzt werden.

## Claims

1. A process for the hydroformylation of at least one olefinically unsaturated compound in the liquid phase in which at least one olefinically unsaturated compound is reacted, in a reaction section, with carbon monoxide and hydrogen in the presence of at least one polar phase comprising:
- a non-aqueous ionic liquid which is liquid at the reaction temperature and at least partly miscible with the reaction products, the ionic liquid comprising at least one salt of general formula Q⁺A⁻, in which Q⁺ represents a quaternary ammonium and/or a quaternary phosphonium and A⁻ represents an anion;
- and at least one catalyst comprising at least one compound or one complex of a transition metal of groups 8, 9 and 10 of the periodic table, optionally co-ordinated with at least one ligand containing at least one atom of phosphorus, arsenic, antimony or nitrogen,
the said process being **characterized in that** after the reaction section at least one organic solvent is added to the medium in a manner such as to improve the separation by demixing of the crude reaction products from the said polar phase, which is recycled to the reaction section for re-use.

2. A process according to claim 1, **characterized in that** it comprises at least the following three sections:
a) at least one section 1, in which the hydroformylation reaction is carried out,
b) at least one section 2, in which are carried out
- mixing of the effluent from the reaction section with at least one organic solvent for the purpose of improving the yield of the products;
- separation of the reaction products and the polar phase containing the ionic liquid and the catalyst;
- and decanting of the polar phase containing the ionic liquid and the catalyst;
c) at least one section 3, in which the reaction products, the organic solvent, which is recycled into the mixing zone of section 2, and the unreacted olefinically unsaturated compound to be hydroformylated, which is recycled to section 1, are separated by distillation.

3. A process according to claim 2, **characterized in that** if the organic solvent used has a boiling point higher than that of the olefinically unsaturated compound to be hydroformylated, the separation of the crude reaction products and the olefinically unsaturated compound to be hydroformylated is first carried out, and then the separation of the final reaction products and the organic solvent.

4. A process according to claim 2, **characterized in that** if the organic solvent used has a boiling point lower that that of the olefinically unsaturated compound to be hydroformylated, the separation of the crude reaction products and the organic solvent is first carried out, and then the separation of the final reaction products from the olefinically unsaturated compound to be hydroformylated.

5. A process according to claim 1 to 4, **characterized in that** the said olefinically unsaturated compound to be hydroformylated is chosen from the group formed by monoolefins, diolefins, conjugated diolefins and olefinic compounds containing one or more heteroatoms, in particular in unsaturated groups.

6. A process according to one of claims 1 to 5, **characterized in that** the organic solvent is chosen from the aliphatic cyclic or acyclic saturated or unsaturated, aromatic or substituted aromatic hydrocarbons.

7. A process according to one of claims 1 to 6, **characterized in that** the organic solvent is the olefinically unsaturated compound to be hydroformylated itself.

8. A process according to one of claims 1 to 7, **characterized in that** in the salt of general formula Q⁺A⁻, the anion A⁻ is chosen such that the salt Q⁺A⁻ is liquid below 90°C.

9. A process according to one of claims 1 to 8, **characterized in that** the anion A⁻ is chosen from the group formed by the following ions: nitrate, sulphate, phosphate, acetate, halogenoacetates, tetrafluoroborate, tetraalkylborates, tetraarylborates, tetrachloroborate hexafluorophosphate, hexafluoroantimonate, fluorosulphonate, perfluoroalkylsulphonates, arene-sulphonates, arene-sulphonates substituted by halogen or halogenoalkyl groups and bis-perfluoroalkylsulphonylamides.

10. A process according to one of claims 1 to 9, **characterized in that** the quaternary ammonium and/or phosphonium cation corresponds to one of general formulae:
NR¹R²R³R⁴⁺ and PR¹R²R³R⁴⁺
or to one of general formulae:
R¹R²N=CR³R⁴⁺ and R¹R²P=CR³R⁴⁺,
in which R¹, R², R³ and R⁴ are identical or different and each represent hydrogen, with the exception of the cation NH₄⁺ for NR¹R²R³R⁴⁺, preferably a single substituent representing hydrogen, or hydrocarbon radicals having 1 to 30 carbon atoms.

11. A process according to one of claims 1 to 9, **characterized in that** the quaternary ammonium and/or phosphonium cation is derived from a nitrogen- and/or phosphorus-containing heterocyclic radical containing 1, 2 or 3 nitrogen and/or phosphorus atoms, in which the rings are made up of 4 to 10 atoms.

12. A process according to one of claims 1 to 7, **characterized in that** the quaternary ammonium and/or phosphonium cation corresponds to one of the formulae:
R¹R²⁺N=CR³-R⁵-R³C=N⁺R¹R² and R¹R²⁺P=CR³-R⁵-R³C=P⁺R¹R²
in which R¹, R² and R³ are identical or different and are as defined in claim 4 and R⁵ represents an alkylene or phenylene radical.

13. A process according to one of claims 1 to 7, **characterized in that** the quaternary ammonium and/or phosphonium cation Q⁺ is chosen form the group formed by N-butylpyridinium, N-ethylpyridinium, pyridinium, 3-ethyl-1-methylimidazolium, 3-butyl-1-methylimidazolium, 1-hexyl-3-methylimidazolium, diethylpyrazolium, N-butyl-N-methylpyrrolidinium, trimethylphenylammonium, tetrabutylphosphonium and tributyl-(tetradecyl)-phosphonium.

14. A process according to one of claims 1 to 13, **characterized in that** the non-aqueous ionic solvent is at least one salt chosen from the group formed by N-butyl-pyridinium hexafluorophosphate, N-ethyl-pyridinium tetrafluoroborate, pyridinium fluorosulphonate, 3-butyl-1-methylimidazolium tetrafluoroborate, 3-butyl-1-methylimidazolium hexafluoroantimonate, 3-butyl-1-methylimidazolium hexafluorophosphate, 3-butyl-1-methylimidazolium trifluoroacetate, 3-butyl-1-methylimidazolium trifluoromethylsulphonate, trimethyl-phenylammonium hexafluorophosphate, 1-hexyl-3-methylimidazolium trifluoroacetate and 3-butyl-1-methylimidazolium bis[(trifluoromethane)sulphonyl]amide.

15. A process according to one of claims 1 to 14, **characterized in that** the transition metal is cobalt, rhodium, iridium, ruthenium, palladium or platinum.

16. A process according to one of claims 1 to 15, **characterized in that** the transition metal compound is a transition metal complex.

17. A process according to one of claims 1 to 16, **characterized in that** the catalytic composition also comprises a ligand or a ligand associated with the transition metal compound, the said ligand being chosen from the group formed by the ligands which carry at least one atom of phosphorus (phosphorus-containing ligands), arsenic or antimony or nitrogen (nitrogen-containing ligands).

18. A process according to claim 17, **characterized in that** the ligand is chosen from tertiary phosphines, poly-phosphines, oxides of phosphines, phosphites, phosphine-phosphite chelates, phosphine phosphine-oxide and the oxide of phosphine-phosphites, arsines, stilbines and nitrogen-containing ligands.

19. A process according to claim 18, **characterized in that** the phosphorus-containing ligand contains at least one amine, ammonium, alcohol, carboxylic acid, sulphonate, phosphonate, ether, phosphonium or sulphonium function.

20. A process according to claim 17, **characterized in that** the nitrogen-containing ligand is chosen from the group formed by monoamines, di-, tri- and polyamines, imines, diimines, pyridines, bipyridines, imidazoles, pyrroles and pyrazoles.

21. A process according to one of claims 1 to 20, **characterized in that** the concentration of the transition metal in the liquid ionic solvent is between 0.1 mmol per litre and 5 mol per litre and the molar ratio between the ligand and the transition metal compound is between 0.1 and 500.

22. A process according to one of claims 1 to 21, **characterized in that** the hydroformylation reaction is carried out with a ratio of the partial pressures of hydrogen to carbon monoxide of 10:1 to 1:10, at a temperature between 30°C and 200°C and under a pressure of between 1 MPa and 20 MPa.

23. A process according to one of claims 1 to 22, **characterized in that** the temperature and/or the pressure during the addition of the solvent are chosen such that they are less than or equal to those used in the reaction section.
